# EUROPEAN PATENT SPECIFICATION

(11) **EP 1 073 455 B1**
(45) Date of publication and mention of the grant of the patent: **08.10.2003**
(21) Application number: 99919857.5
(22) Date of filing: 15.04.1999
(51) Int. Cl.: A61K 38/16, A61K 38/18, A61K 38/30, A61K 38/17, A61K 38/39, A61K 31/70

(54) **COMPOSITIONS AND METHODS FOR EXTENDING THE ACTION OF CLOSTRIDIAL NEUROTOXIN**
ZUSAMMENSETZUNGEN UND METHODE ZUM VERLÄNGUNG DER WIRKUNG DES CLOSTRIDIUMSNEUROTOXINS
COMPOSITIONS ET PROCEDES PERMETTANT D'ETENDRE L'ACTION D'UNE NEUROTOXINE CLOSTRIDIALE

(30) Priority: 29.04.1998 US 83472 P
(43) Date of publication of application: 07.02.2001
(73) Proprietor: Allergan, Inc., Irvine, CA 92612 (US)
(72) Inventor: DOLLY, J., Oliver, Cheam Surrey SM2 7LP (GB); AOKI, Kei, Roger, Coto de Caza, CA 92679 (US); DE PAIVA, Anton, London W5 5EG (GB)
(74) Representative: HOFFMANN - EITLE
(86) International application number: US9908303
(87) International publication number: WO99055359

(56) References cited:
- US-A- 5 721 215
- CARONI P. ET AL: "Signaling by insulin-like growth factors in paralyzed skeletal muscle: Rapid induction of IGF1 expression in muscle fibers and prevention of interstitial cell proliferation by IGF-BP5 and IGF-BP4" THE JOURNAL OF NEUROSCIENCE, vol. 14, no. 5, 1994, pages 3378-3388, XP002115584 cited in the application
- CIFUENTES-DIAZ C.: "The peripheral nerve and the neuromuscular junction are affected in the tenascin-C deficient mouse" CELLULAR AND MOLECULAR BIOLOGY, vol. 44, no. 2, March 1998 (1998-03), pages 357-379, XP002115585
- BOOTH C.M. ET AL: "An antibody to neural cell adhesion molecule impairs motor nerve terminal sprouting in a mouse muscle locally paralysed with botulinum toxin" NEUROSCIENCE, vol. 35, no. 1, 1990, pages 85-91, XP002115586
- GURNEY M.E. ET AL: "Induction of motor neuron sprouting in vivo by ciliary neurotrophic factor and basic fibroblast growth factor" THE JOURNAL OF NEUROSCIENCE, vol. 12, no. 8, 1992, pages 3241-3247, XP002115587 cited in the application
- A. K. MCALLISTER ET AL: "Neurotrophin regulation of cortical dendritic growth requires activity" NEURON , vol. 17, 1996, pages 1057-1064, XP002115588 cited in the application
- CARONI P. ET AL: "Role of muscle insulin-like growth factors in nerve sprouting: suppression of terminal sprouting in paralyzed muscle by IGF-binding protein 4" THE JOURNAL OF CELL BIOLOGY, vol. 125, no. 4, 1994, pages 839-902, XP002115589 cited in the application
- PAIVA, A. ET AL: "Functional repair of motor endplates after botulinum neurotoxin type A poisoning: Biphasic switch of synaptic activity between nerve sprouts and their parent terminals" PROCEEDINGS OF THE NATIONAL ACADEMY OF SCIENCES, USA, vol. 96, March 1999 (1999-03), pages 3200-3205, XP002115590 cited in the application

## Description

### Field of the Invention

The present invention is directed towards compositions for inhibiting neural sprouting in neurons that have been subjected to botulinum toxin. Also disclosed are compositions for extending the period of time during which treatment of nerve cells with botulinum toxin is effective to prevent innervation of a cell or tissue, such as muscle cells or tissue. Such compositions are effective in the treatment of spasms or muscular tetanus. Disclosed as well are compositions for stimulating neural outgrowth.

### Background of the invention

Neurotoxins, such as those obtained from *Clostridium botulinum* and *Clostridium tetanus,* are highly potent and specific poisons of neural cells. These Gram positive bacteria secrete two related but distinct toxins, each comprising two disulfide-linked amino acid chains: a light chain (L) of about 50 KDa and a heavy chain (H) of about 100 KDa, which are wholly responsible for the symptoms of these diseases.

The tetanus and botulinum toxins are among the most lethal substances known to man, having a lethal dose in humans of between 0.1 ng and 1 ng per kilogram of body weight. Tonello et al., *Adv. Exp. Med. & Biol.* 389:251-260 (1996). Both toxins function by inhibiting neurotransmitter release in affected neurons. The tetanus neurotoxin (TeNT) acts mainly in the central nervous system, while botulinum neurotoxin (BoNT) acts at the neuromuscular junction by inhibiting acetylcholine release from the axon of the affected neuron into the synapse, resulting in a localized flaccid paralysis. The effect of intoxication on the affected neuron is long-lasting and has been thought to be irreversible.

The tetanus neurotoxin (TeNT) is known to exist in one immunologically distinct type; the botulinum neurotoxins (BoNT) are known to occur in seven different immunogenic types, termed BoNT/A through BoNT/G. While all of these types are produced by isolates of *C. botulinum,* two other species, *C. baratii* and *C. butyricum* also produce toxins similar to /F and /E, respectively. *See* e.g., Coffield et al., *The Site and Mechanism of Action of Botulinum Neurotoxin* in *Therapy with Botulinum Toxin* 3-13 (Jankovic J. & Hallett M. eds. 1994), the disclosure of which is incorporated herein by reference.

Regardless of type, the molecular mechanism of intoxication appears to be similar. In the first step of the process, the toxin binds to the presynaptic membrane of the target neuron through a specific interaction between the heavy chain and a cell surface receptor; the receptor is thought to be different for each type of botulinum toxin and for TeNT. The carboxy terminus of the heavy chain appears to be important for targeting of the toxin to the cell surface.

In the second step, the toxin crosses the plasma membrane of the poisoned cell. The toxin is first engulfed by the cell through receptor-mediated endocytosis, and an endosome containing the toxin is formed. The toxin then escapes the endosome into the cytoplasm of the cell. This last step is thought to be mediated by the amino terminus of the heavy chain, which triggers a conformational change of the toxin in response to a pH of about 5.5 or lower. Endosomes are known to possess a proton pump which decreases intra-endosomal pH. The conformational shift exposes hydrophobic residues in the toxin, which permits the toxin to embed itself in the endosomal membrane. The toxin then translocates through the endosomal membrane into the cytosol.

The last step of the mechanism of botulinum toxin activity appears to involve reduction of the disulfide bond joining the heavy and light chain. The entire toxic activity of botulinum and tetanus toxins is contained in the light chain of the holotoxin; the light chain is a zinc (Zn++) endopeptidase which selectively cleaves proteins essential for recognition and docking of neurotransmitter-containing vesicles with the cytoplasmic surface of the plasma membrane, and fusion of the vesicles with the plasma membrane. TxNT, BoNT/B BoNT/D, BoNT/F, and BoNT/G cause degradation of synaptobrevin 2 (also called vesicle-associated membrane protein (VAMP)), a synaptosomal membrane protein. Most of the VAMP present at the cytosolic surface of the synaptic vesicle is removed as a result of any one of these cleavage events. Each toxin specifically cleaves a different bond.

BoNT/A and /E selectively cleave the plasma membrane-associated protein SNAP-25; this protein is bound to and present on the cytosolic surface of the plasma membrane. BoNT/C cleaves syntaxin, an integral protein having most of its mass exposed to the cytosol. Syntaxin interacts with the calcium channels at presynaptic terminal active zones. *See* Tonello et al., *Tetanus and Botulism Neurotoxins in Intracellular Protein Catabolism* 251-260 (Suzuki K & Bond J. eds. 1996), the disclosure of which is incorporated by reference as part of this specification. BoNT/C₁ also cleaves SNAP-25 at a peptide bond next to that cleaved by BoNT/A.

Both TeNT and BoNT are taken up at the neuromuscular junction. BoNT remains within peripheral neurons, and blocks release of the neurotransmitter acetylcholine from these cells. Through its receptor, TeNT enters vesicles that move in a retrograde manner along the axon to the soma, and is discharged into the intersynaptic space between motor neurons and the inhibitory neurons of the spinal cord. At this point, TeNT binds receptors of the inhibitory neurons, is again internalized, and the light chain enters the cytosol to block the release of the inhibitory neurotransmitters 4-aminobutyric acid (GABA) and glycine from these cells. Id.

Because of its specifically localized effects, dilute preparations of BoNT have been used since 1981 as therapeutic agents in the treatment of patients having various spastic conditions, including strabismus (misalignment of the eye), bephlarospasm (involuntary eyelid closure) and hemifacial spasm. *See* e.g., Borrodic et al., *Pharmacology and Histology Botulinum Toxin* in *Therapy with Botulinum Toxin* 3-13 (Jankovic J. & Hallett M. eds. 1994), incorporated by reference herein. Of the seven toxin types, BoNT/A is the most potent of the BoNTs, and the most well characterized. Intramuscular injection of spastic tissue with dilute preparations of BoNT/A has been also used effectively to treat spasticity due to brain injury, spinal cord injury, stroke, multiple sclerosis and cerebral palsy. The extent of paralysis depends on both the dose and dose volume delivered to the target site. Typically, the neurotoxin is administered in a preparation that also contains several non-toxic proteins as well, including hemagglutins and associated glycoproteins that assist in maximizing its stability and presentation to the target motor neuron.

Typically there is a 24 to 72 hour delay between the administration of the toxin and the onset of the clinical effect. Exposure to the toxin causes denervation atrophy. *See* e.g., Dutton J., *Acute and Chronic Effects of Botulinum* *Toxin in the Management of Blepharospasm,* in *Therapy with Botulinum Toxin* at 199, incorporated by reference herein. Although the therapeutic application of BoNT has been extraordinarily effective, what side effects have been observed are mainly immediate effects associated with the treatment event itself. Peripheral effects causing weakness in adjacent muscles may sometimes occur; these effects usually do not persist beyond 1 to two weeks. The specific manifestations of these effects upon adjacent muscle groups depend upon the particular indication being treated. For example, patients treated for blepharospasm sometimes experience ptosis, and swallowing problems may occur after injection of neck muscles for torticollis.

Other possible consequences of treatment include the potential for overdosage through miscalculation or differences in activity between different preparations of the toxin, generalized fatigue, and the potential for allergic reactions.

A feature of treatment with BoNT/A, and other clostridial neurotoxin types, is that the paralytic action is temporary with symptoms reappearing in patients within a few months after toxin injection. This characteristic has been thought to be associated with the observed sprouting of nascent, synaptically active processes at the neuromuscular junction (NMJ). The production of such sprouts following BoNT/A therapeutic treatment has appeared to contribute to the reinervation of the treated tissue and therefore the need for repeated serial injections of the toxin.

The duration of paralytic action caused by clostridial. neurotoxin and the extent of sprouting, appears to depend on the neurotoxin subtype studied and therefore appears to be related to the specific SNARE target cleaved by each neurotoxin. Thus, BoNT/A and BoNT/C₁, which cleave the t-SNARE protein SNAP-25 within one amino acid of each other, cause a long lasting paralysis with a long average sprout length observed. BoNT/F, which cleaves the v-SNARE protein VAMP, causes a paralysis of shorter duration and sprouts of shorter average length. BoNT/E, which cleaves SNAP-25 at a different position than that of BoNT/A and BoNT/C₁ (thereby liberating a SNAP fragment of different size from the plasma membrane), has a short duration period of about 5 days, and virtually no neural sprouting is observed. Without wishing to be bound by theory, these observations suggest that neural sprouting and duration of paralysis are normally related events, and that the cleavage products of BoNT proteolytic digestion (i.e., the liberated fragment or the membrane-bound fragment) can directly or indirectly regulate, or be coregulated with, neural sprouting.

It would therefore be advantageous to design a method whereby the sprouting phenomenon may be uncoupled from duration of therapeutic effect, and thus delayed, blocked or attenuated so as to prolong the effects of injection of tissue with toxin. Although the experiments described herein utilize a BoNT/A preparation, it will be recognized by those of skill in the art that the methods shown herein will be suitable for employment using other clostridial toxins, such as BoNT/B through /G, and TeNT, in which a sprouting pathway can be observed.

Additionally, it would be advantageous to provide herein compositions effective for the inhibition or prevention of the sprouting phenomenon. Such compositions and methods would lessen the need for patients to undergo repeated neurotoxin treatment.

### Summary of the Invention

The present invention concerns compositions for increasing the period of time between therapeutic treatments of neural tissue with a clostridial neurotoxin; thus allowing to increase the effectiveness of such treatments. A direct advantage of such compositions is an increased therapeutic "life" and a concomitant lessening in the required frequency of treatment of the patient with neurotoxin. Reducing frequency of treatment would provide less opportunity for a patient to experience the side effects described above that are observed following treatment, but which tend to subside long before the effectiveness of the toxin in the target area has subsided. Additionally, reduced frequency of treatment provides less opportunity for miscalculation of dosage amount and other treatment-specific risks.

Accordingly, an aspect of the invention concerns a composition comprising a first agent comprising a clostridial neurotoxin for use as a therapeutic agent and a second agent able to extend the duration of therapeutic . benefit of said first agent, wherein the second agent is effective to attenuate the production of nerve terminal sprouts following treatment of a neuromuscular junction with the clostridial neurotoxin.

In a particular embodiment of this aspect of the invention, the first and second agent may comprise a single entity which is provided the patient in a single treatment session. For example, the entity may comprise a single molecule, or a disulfide-linked multichain polypeptide. Additionally or alternatively, the entity may comprise one or more adsorbed or linked heterogroup, such as a small organic molecule or a nucleic acid linked thereto. The entity preferably comprises both the receptor binding and translocation activities of a clostridial heavy chain and an active portion of a clostridial toxin light chain. The light chain may also comprise an auxiliary enzymatic activity, such as a ribonuclease, which specifically cleaves a nucleic acid encoding an intraneuronal factor which is responsible for the expression, activation and/or secretion of neurotrophic factors or cell adhesion molecules. In a preferred embodiment, such an auxiliary activity is provided by a ribozyme. By ribozyme is meant a nucleic acid or nucleic acid analog having a sequence-specific nuclease activity; the construction and use of ribozymes are well known in the art; see e.g., Cech, T., *Science* 236:1532-1539(1987); Cech, T. R., *Curr. Opin. Struct. Biol.* 2:605-609 (1992); and Usman et al., *Nucleic Acids & Mol. Biol.* 10:243-264 (1996), the disclosures of which are hereby incorporated by reference herein. By nucleic acid analog is meant a polymeric molecule able to form a sequence-specific hybrid with a target single-stranded nucleic acid; such analogs may contain modified nucleotides (or ribonucleotides) such as 3'-O methyl nucleotides, phosphorothioate modified nucleotides, methylphosphonate nucleotides, or nucleotide bases separated by a peptide-like bond.

Alternatively, a nucleic acid or nucleic acid analog comprised in the single entity referred to above may be an antisense agent able to selectively bind to a nucleic acid encoding an intraneuronal factor which is responsible for the expression, activation and/or secretion of neurotrophic factors or cell adhesion molecules. This antisense agent may further provide a double-stranded substrate for the action of an intracellular RNAse H activity. Details concerning certain embodiments of these aspects of the invention are contained in *e.g.*, Dolly et al., International Publication No. WO95/327 entitled *Modification of Clostridial Toxins for Use as Transport Proteins* and Uherek et al., *J. Biol. Chem.* 273:8835-8841 (1998). These two references are incorporated by reference as part of the present application.

A nucleic acid moiety linked polypeptide portion of the entity may encode a protein or polypeptide having the ability to be expressed within a neuron and to directly or indirectly regulate the expression, activation and/or secretion of neurotrophic factors or cell adhesion molecules.

In preferred aspects of the invention, the second agent is selected from the group consisting of agents able to compete with, down-regulate, or neutralize the effects of: IGFI, IGF II, a neurotrophic factor, leukemia inhibitory factor, a nerve cell adhesion molecule and neural agrin. In a more preferred aspect of the invention, the neurotrophic factor is selected from the group consisting of: ciliary neurotrophic factor, NT-3, NT-4, and brain-derived neurotrophic factor and/or the nerve cell adhesion molecule is selected from the group consisting of tenascin-C, ninjurin, neural cell adhesion molecule.

Applicants have surprisingly discovered that recovery of neural function following poisoning of nerve terminals with clostridial neurotoxin involves two distinct and apparently coordinated events. First, the poisoned endplate becomes synaptically inactive. Shortly thereafter the endplate elaborates thin nascent axon neural processes. These processes or "sprouts" are synaptically competent after about 14 days following treatment with clostridial neurotoxin. The sprouts continue growing, reaching a maximal length and level of complexity after about 42 days following treatment with neurotoxin. During this time, the endplate remains synaptically inactive.

Secondly, after about 42 days, the sprouts begin to regress, shortening in length and decreasing in complexity. At the same time, the original endplate begins to become synaptically active, undergoing synaptic vesicle turnover. The increase in such turnover reaches that of the unpoisoned endplate after about 91 days post-treatment, at approximately the same time that the sprouting phenomenon has completely regressed, and no sprouts can be observed. These findings are reported in DePaiva et al., *Proc. Nat'1. Acad. Sci.* 96:3200-3205 (March 1999), which is hereby incorporated by reference herein.

These observations are diametrically opposed to the prevailing wisdom in the art, in which it has largely been assumed that the original endplate is permanently inactivated upon treatment with clostridial toxin, and that all return of synaptic activity is due to extension of sprouts to compensate for the lack of a neurologically functional endplate. However, as indicated, the old paradigm has been shown by the present Applicants to be in error, in that the poisoned endplate regains neurological function over time, while the axon sprouts regress so that after a given time period the nerve terminal appears essentially as it did prior to treatment. Therefore, Applicants have discovered that, far from being a permanent feature, the axonal sprouts assume a temporary role in the rehabilitation of the poisoned endplate.

Although not wishing to be limited by theory, Applicants believe that these results indicate that the two events outlined above are temporally coordinated, in that a blockage of the neural sprouting phenomenon would delay or block the recovery of the functional endplate. Such temporal coordination could be due to the secretion of one or more factor by the damaged neural endplate (or the inactive muscle fiber) that has neurotrophic effects resulting in the formation of neural sprouts; these sprouts may then elaborate a factor (either the same or different from the first factor) which promotes continued sprouting. This factor may be produced during neurite sprouting in amounts sufficient for the reinervation of the neurotoxin-damaged endplate even after treatment with clostridial toxin. Thus, treatment of cells with an agent able to block neural sprouting would also delay or otherwise attenuate the ability of the treated endplate to experience return of neurological function, and in fact may well block such return altogether.

As indicated, the signaling event indicating the initiation of the neural sprouting phenomenon appears to be mediated by a cytokine or other intercellular messenger. One such agent, agrin, appear to be an important player, if not the key molecule, in the formation of the neuromuscular junction in development, and in neuromuscular regeneration. *See* Ruegg M.A. and Bixby J. L., *Trends in Neurol. Sci.* 21:22-27 (1998), the disclosure of which is incorporated herein by reference. Agrin appears to be present in a number of isoforms, which result from alternative mRNA splicing. Soluble agrin isolated from synaptic basal lamina extracts (to which it binds following secretion) is able to induce the aggregation of acetylcholine receptors in the postsynaptic portions of muscle cells. Agrin present in motor neuron terminals (n-agrin) contains an insert, relative to other agrin species, in a region termed the B/_{z}. region; this insert is important in conferring the ability on n-agrin to aggregate acetylcholine receptors in postsynaptic tissue. Neural agrin is released by the motor-nerve terminal and is believed by Applicants to induce *post*synaptic specialization and up-regulation of other factors, such as muscle-diffusable factors, involved in the neural sprouting response.

It is anticipated that compositions which interfere with the sprouting phenomenon (such as by the preventing the action of agrin) would delay the return of innervation to cells which are controlled by neurons which have been therapeutically poisoned (i.e., by BoNT or TeNT) or otherwise damaged. This is because, as indicated above, the rate of return of neural function appears to be partly dependent upon the presence of synaptically active sprouts. Additionally, agents to be used in the inhibition of sprouting may very well also delay or prevent the recovery of neural activity of the endplate following poisoning.

Thus utilization of such compositions would therefore be expected to extend the effective period of treatment of tissue with a clostridial toxin, by delaying the regeneration of active neuromuscular synapses, both through inhibition of sprouting and of recovery of the poisoned endplate.

### Detailed Description of the Invention

This invention is drawn to compositions for increasing the therapeutic effectiveness of treatment of tissue with clostridial neurotoxin. This increase in effectiveness is made possible by the surprising discovery that regeneration of neural tissue damaged by treatment with clostridial neurotoxin is a complex occurrence in which two coordinated events take place.

In the first of these events, the poisoned neuromuscular endplate becomes synaptically inactive, demonstrating no exocytosis of synaptic vesicles and thus no transport of intracellular acetylcholine. In four days after treatment, the endplate begins to form neural sprouts that are shown to release and regenerate synaptic vesicles. These sprouts grow in length and complexity until approximately 42 days following treatment with the neurotoxin; at this point the neural sprouts begin to regress and shorten. At ninety-one days following treatment, the neural sprouts can no longer be seen.

In the second event, simultaneously with the beginning of regression of the neural sprouts, the synaptically inactive endplate begins to regain the ability to release acetylcholine and begin to recycle synaptic vesicles. This ability, which begins at relatively low levels, increases over the time period indicated above. At approximately 91 days following treatment with clostridial neurotoxin the endplate is histologically and synaptically indistinguishable from the condition of the endplate before treatment with clostridial neurotoxin.

These findings indicate that one may therapeutically . intervene at one of the major steps of the sprouting phenomenon to prevent or attenuate the neural sprouting as a method of extending the effective period during which tissue treated with the toxin remains paralyzed. In an initial step, the muscle cells surrounding the neural endplate respond, either sensing the inactive muscle or in response to a signal from the poisoned nerve terminal, by producing muscle-derived diffusable factors. The expression of a number of muscle-derived signaling factors appears to be upregulated by muscle inactivation; such factors include insulin-like growth factors (IGF-1 and IGF-2). A factor such as neural agrin is believed to be the initial signal directing the muscle cell to produce the IGF molecules. Reports have demonstrated that IGF I and IGF II effect neurite outgrowth in cultured BoNT/A treated dorsal root ganglia, and also are able to stimulate the initial sprouting response in paralyzed mouse gluteus muscle. *See* Caroni, P. and Schneider, C. *J. Neurosci.* 14:3378-3388 (1994) and Caroni, P., et al. *J. Cell Biol.* 125:893-902 (1994).

Thus, blocking the effects of such muscle derived diffusable factors that positively affect neurite outgrowth and sprouting may attenuate not only clostridial neurotoxin-induced sprouting, but may also delay the eventual recovery of neurotransmission at the poisoned nerve terminals. Such blocking may occur through the use of antibodies specific for the muscle derived diffusable factor in question, or that are common to such muscle derived diffusable factors.. Alternatively, there are naturally occurring binding proteins, such as the IGF binding proteins IGF-BP 4 and IGF-BP 5, which can bind to, and therefore block, the neurotrophic effect of such diffusable factors.

IGF-BP 4 has an amino acid sequence (from the amino terminus) of: 
MLPLCLVAALLLAAGPGPSLGDEAIHCPPCSEEKLARCRPPVGCEELVREPGCGCCATCA
LGLGMPCGVYTPRCGSGLRCYPPRGVEKPLHTLMHGQGVCMELAEIEAIQESLQPSDKDE
GDHPNNSFSPCSAHDRRCLQKHFAKIRDRSTSGGKMKVNGAPREDARPVPQGSCQSELHR
ALERLAASQSRTHEDLYIIPIPNCDRNGNFHPKQCHPALDGQRGKCWCVDRKTGVKLPGG
LEPKGELDCHQLADSFRE

(SEQ ID NO: 1)
 This is encoded within the nucleotide base sequence (from 5' to 3') of:

GTGCCCTCCG CCGCTCGCCC GCGCGCCCGC GCTCCCCGCC TGCGCCCAGC
GCCCCGCGCC CGCGCCCCAG TCCTCGGGCG GTCATGCTGC CCCTCTGCCT
CGTGGCCGCC CTGCTGCTGG CCGCCGGGCC CGGGCCGAGC CTGGGCGACG
AAGCCATCCA CTGCCCGCCC TGCTCCGAGG AGAAGCTGGC GCGCTGCCGC
CCCCCCGTGG GCTGCGAGGA GCTGGTGCGA GAGCCGGGCT GCGGCTGTTG
CGCCACTTGC GCCCTGGGCT TGGGGATGCC CTGCGGGGTG TACACCCCCC
GTTGCGGCTC GGGCCTGCGC TGCTACCCGC CCCGAGGGGT GGAGAAGCCC
CTGCACACAC TGATGCACGG GCAAGGCGTG TGCATGGAGC TGGCGGAGAT
CGAGGCCATC CAGGAAAGCC TGCAGCCCTC TGACAAGGAC GAGGGTGACC
ACCCCAACAA CAGCTTCAGC CCCTGTAGCG CCCATGACCG CAGGTGCCTG
CAGAAGCACT TCGCCAAAAT TCGAGACCGG AGCACCAGTG GGGGCAAGAT
GAAGGTCAAT GGGGCGCCCC GGGAGGATGC CCGGCCTGTG CCCCAGGGCT
CCTGCCAGAG CGAGCTGCAC CGGGCGCTGG AGCGGCTGGC CGCTTCACAG
AGCCGCACCC ACGAGGACCT CTACATCATC CCCATCCCCA ACTGCGACCG
CAACGGCAAC TTCCACCCCA AGCAGTGTCA CCCAGCTCTG GATGGGCAGC
GTGGCAAGTG CTGGTGTGTG GACCGGAAGA CGGGGGTGAA GCTTCCGGGG
GGCCTGGAGC CAAAGGGGGA GCTGGACTGC CACCAGCTGG CTGACAGCTT
TCGAGAGTGA GGCCTGCCAG CAGGCCAGGG ACTCAGCGTC CCCTGCTACT
CCTGTGCTCT GGAGGCTGCA GAGCTGACCC AGAGTGGAGT CTGAGTCTGA

GTCCTGTCTC TGCCTGCGGC CCAGAAGTTT CCCTCAAATG CGCGTGTGCA
CGTGTGCGTG TGCGTGCGTG TGTGTGTGTT TGTGAGCATG GGTGTGCCCT
TGGGGTAAGC CAGAGCCTGG GGTGTTCTCT TTGGTGTTAC ACAGCCCAAG
AGGACTGAGA CTGGCACTTA GCCCAAGAGG TCTGAGCCCT GGTGTGTTTC
CAGATCGATC CTGGATTCAC TCACTCACTC ATTCCTTCAC TCATCCAGCC
ACCTAAAAAC ATTTACTGAC CATGTACTAC GTGCCAGCTC TAGTTTTCAG
CCTTGGGAGG TTTTATTCTG ACTTCCTCTG ATTTTGGCAT GTGGAGACAC
TCCTATAAGG AGAGTTCAAG CCTGTGGGAG TAGAAAAATC TCATTCCCAG
AGTCAGAGGA GAAGAGACAT GTACCTTGAC CATCGTCCTT CCTCTCAAGC
TAGCCAGAGG GTGGGAGCCT AAGGAAGCGT GGGGTAGCAG ATGGAGTAAT
GGTCACGAGG TCCAGACCCA CTCCCAAAGC TCAGACTTGC CAGGCTCCCT
TTCTCTTCTT CCCCAGGTCC TTCCTTTAGG TCTGGTTGTT GCACCATCTG
CTTGGTTGGC TGGCAGCTGA GAGCCCTGCT GTGGGAGAGC GAAGGGGGTC
AAAGGAAGAC TTGAAGCACA GAGGGCTAGG GAGGTGGGGT ACATTTCTCT
GAGCAGTCAG GGTGGGAAGA AAGAATGCAA GAGTGGACTG AATGTGCCTA
ATGGAGAAGA CCCACGTGCT AGGGGATGAG GGGCTTCCTG GGTCCTGTTC
CCTACCCCAT TTGTGGTCAC AGCCATGAAG TCACCGGGAT GAACCTATCC
TTCCAGTGGC TCGCTCCCTG TAGCTCTGCC TCCCTCTCCA TATCTCCTTC
CCCTACACCT CCCTCCCCAC ACCTCCCTAC TCCCCTGGGC ATCTTCTGGC
TTGACTGGAT GGAAGGAGAC TTAGGAACCT ACCAGTTGGC CATGATGTCT TTTCT

(SEQ ID NO: 3)

IGFBP 5 has an amino acid sequence (from the amino terminus) of: 
MVLLTAVLLLLAAYAGPAQSLGSFVHCEPCDEKALSMCPPSPLGCELVKEPGCGCCMTCA
LAEGQSCGVYTERCAQGLRCLPRQDEEKPLHALLHGRGVCLNEKSYREQVKIERDSREHE
EPTTSEMAEETYSPKIFRPKHTRISELKAEAVKKDRRKKLTQSKFVGGAENTAHPRIISA
PEMRQESEQGPCRRHMEASLQELKASPRMVPRAVYLPNCDRKGFYKRKQCKPSRGRKRGI
CWCVDKYGMKLPGMEYVDGDFQCHTFDSSNVE

(SEQ ID No. 2)
 and is encoded within a nucleotide base sequence (from 5' to 3') of:

GGGGAAAAGA GCTAGGAAAG AGCTGCAAAG CAGTGTGGGC TTTTTCCCTT
TTTTTGCTCCT TTTCATTAC CCCTCCTCCG TTTTCACCCT TCTCCGGACT
TCGCGTAGAA CCTGCGAATT TCGAAGAGGA GGTGGCAAAG TGGGAGAAAA

GAGGTGTTAG GGTTTGGGGT TTTTTTGTTT TTGTTTTTGT TTTTTAATTT
CTTGATTTCA ACATTTTCTC CCACCCTCTC GGCTGCAGCC AACGCCTCTT
ACCTGTTCTG CGGCGCCGCG CACCGCTGGC AGCTGAGGGT TAGAAAGCGG
GGTGTATTTT AGATTTTAAG CAAAAATTTT AAAGATAAAT CCATTTTTCT
CTCCCACCCC CAACGCCATC TCCACTGCAT CCGATCTCAT TATTTCGGTG
GTTGCTTGGG GGTGAACAAT TTTGTGGCTT TTTTTCCCCT ATAATTCTGA
CCCGCTCAGG CTTGAGGGTT TCTCCGGCCT CCGCTCACTG CGTGCACCTG
GCGCTGCCCT GCTTCCCCCA ACCTGTTGCA AGGCTTTAAT TCTTGCAACT
GGGACCTGCT CGCAGGCACC CCAGCCCTCC ACCTCTCTCT ACATTTTTGC
AAGTGTCTGG GGGAGGGCAC CTGCTCTACC TGCCAGAAAT TTTAAAACAA
AAACAAAAAC AAAAAAATCT CCGGGGGCCC TCTTGGCCCC TTTATCCCTG
CACTCTCGCT CTCCTGCCCC ACCCCGAGGT AAAGGGGGCG ACTAAGAGAA
GATGGTGTTG CTCACCGCGG TCCTCCTGCT GCTGGCCGCC TATGCGGGGC
CGGCCCAGAG CCTGGGCTCC TTCGTGCACT GCGAGCCCTG CGACGAGAAA
GCCCTCTCCA TGTGCCCCCC CAGCCCCCTG GGCTGCGAGC TGGTCAAGGA
GCCGGGCTGC GGCTGCTGCA TGACCTGCGC CCTGGCCGAG GGGCAGTCGT
GCGGCGTCTA CACCGAGCGC TGCGCCCAGG GGCTGCGCTG CCTCCCCCGG
CAGGACGAGG AGAAGCCGCT GCACGCCCTG CTGCACGGCC GCGGGGTTTG
CCTCAACGAA AAGAGCTACC GCGAGCAAGT CAAGATCGAG AGAGACTCCC
GTGAGCACGA GGAGCCCACC ACCTCTGAGA TGGCCGAGGA GACCTACTCC
CCCAAGATCT TCCGGCCCAA ACACACCCGC ATCTCCGAGC TGAAGGCTGA
AGCAGTGAAG AAGGACCGCA GAAAGAAGCT GACCCAGTCC AAGTTTGTCG
GGGGAGCCGA GAACACTGCC CACCCCCGGA TCATCTCTGC ACCTGAGATG
AGACAGGAGT CTGAGCAGGG CCCCTGCCGC AGACACATGG AGGCTTCCCT
GCAGGAGCTC AAAGCCAGCC CACGCATGGT GCCCCGTGCT GTGTACCTGC
CCAATTGTGA CCGCAAAGGA TTCTACAAGA GAAAGCAGTG CAAACCTTCC
CGTGGCCGCA AGCGTGGCAT CTGCTGGTGC GTGGACAAGT ACGGGATGAA
GCTGCCAGGC ATGGAGTACG TTGACGGGGA CTTTCAGTGC CACACCTTCG
ACAGCAGCAA CGTTGAGTGA TGCGTCCCCC CCCAACCTTT CCCTCACCCC
CTCCCACCCC CAGCCCCGAC TCCAGCCAGC GCCTCCCTCC ACCCCAGGAC
GCCACTCATT TCATCTCATT TAAGGGAAAA ATATATATCT ATCTATTTGA
GGAAAAAAAA AAAAAAAAAA AA

(SEQ ID NO: 4)

These binding proteins can be made synthetically or cloned and produced for therapeutic purposes, while a cell line producing a desired monoclonal antibody can be maintained for relatively large-scale antibody production. Cloning and general antibody methodologies are commonplace in the art; such methodologies are disclosed within Sambrook et al., *Molecular cloning: A Laboratory Manual* (2nd ed. Cold Spring Harbor Laboratory Press 1989), the disclosure of which is hereby incorporated by reference as part of this disclosure.

Rather than using competitive methods, another aspect of the invention involves the use of a cholinergic special transporter to insert a gene which produces inactive receptors for one or more factor involved in promotion of neural sprouting. Such receptors would maintain high specific binding constants to their ligands, but the biological activity of the receptors would be abrogated; such receptors could be easily be generated and screened through the introduction of mutations in the nucleotide sequence encoding the protein, and assaying the mutants for binding strength and biological activity. Alternatively, the neurotrophic activities of factors produced by the neural endplate or nascent sprouts may be inhibited through intracellular targeting and delivery of a competitive inhibitor, ribozyme, transcriptional suppresser or other agent specifically able to block or attenuate such activities, as described above. Such intracellular targeting is disclosed in references such as, e.g., Dolly et al., International Patent Publication No. WO95/32738, previously incorporated by reference herein.

The second point at which intervention in the sprouting phenomenon may be made is during the stage of axonal outgrowth and arbor development. At this stage such outgrowth has already been initiated, but auxiliary factors. appear to be necessary in order to maintain axonal growth. A number of factors are known to affect the rates of outgrowth, and may also effect the survivability of many types of neurons. Such factors include ciliary neurotrophic factor (CNTF); neurotrophins, including NT-3, NT-4, and brain-derived neurotrophic factor (BDNF); and leukemia inhibitory factor (LIF). Not only are these factors important in establishing an initial rate of axonal outgrowth, but they appear to either directly or indirectly stimulate their own production -- therefore blocking the initial outgrowth of sprouts may be essential in preventing further propagation through the continued expression of such factors.

The same methods as disclosed above can be used to prevent one or more of the agents listed above to manifest its activity. As would be expected by those of skill in the art in light of the present disclosure, such agents, destroy or bind to, and therefore attenuate the activity of these factors or their block their ability to bind to their receptors, or inactive forms of their receptors, would be useful when used in conjunction with a clostridial neurotoxin to prevent or reduce the rate of sprouting of neurites from the poisoned or damaged endplate.

The third stage at which the sprouting phenomenon may be attenuated or inhibited concerns the binding of axons to the extracellular matrix. Axons are guided to cellular processes containing the appropriate neurotransmitter receptors by binding to components of the extracellular matrix. Such binding involves a variety of cell-borne or matrix associated adhesion molecules. Tenascin-C is an extracellular matrix component derived from Schwann cells that appears to bind neural processes. Ninjurin is a cell surface adhesion molecule that is up-regulated following peripheral nerve injury and thought to be involved in exonal guidance. *See* Araki, T., et al., *J. Biol. Chem*. **272**:21373-21380 (1997), incorporated by reference herein. Similarly, neural-cell adhesion molecule (N-CAM), is an adhesion molecule which is thought to be involved in binding of neural sprouts to the extracellular matrix. The same techniques indicated above may be employed to prevent the expression or inhibit the activity of these molecules when used as part of a clostridial neurotoxin therapy.

Thus, in one aspect the present invention is drawn to compositions for extending the effective period during which tissue treated with clostridial toxin is paralyzed. The agent able to prevent the neuroregenitive activity of a polypeptide is selected from the group consisting of IGF-1, IGF-2, cilary neurotrophic factor, NT-3, NT-4, brain-derived neurotrophic factor, leukemia inhibitory factor, tenascin-C, ninjurin, neural cell adhesion molecule, and neural agrin.

In one preferred embodiment, the agent comprises a polypeptide able to bind to IGF-1 and/or IGF-2 in a manner that prevents an IGF molecule from binding to or activating a cell surface receptor involved in the initiation of neural sprouting. In a most preferred embodiment the polypeptide comprises at least a portion of a amino acid sequence selected from the group consisting of: IGFBP-4 (SEQ ID NO: 1) or IGFBP5 (SEQ ID NO: 2). Preferably said portion comprises at least 10 contiguous amino acids of said sequence; more preferably said portion comprises at least 20 contiguous nucleotides of said sequence. Most preferably, the portion comprises an amino acid sequence selected from the group consisting of the entire amino acid sequence of IGFBP-4 or IGFBP-5.

Treatment of cells with such a composition may be accomplished either before or simultaneously with treatment with clostridial toxin. Preferably, the clostridial toxin is a botulinum toxin. Even more preferably, the botulinum toxin comprises BoNT/A. In other embodiments, the clostridial toxin is TeNT. Agent which are able to bind to any of these factors in a manner that inhibits their neurotrophic activity, or which bind to the receptors for such factors, would, in light of the present application, be expected to function as agents for extending the effective period between treatments of tissue with a neurotoxin.

Another embodiment comprises a cholinergic specific transporter joined to a gene encoding a gene which produces an inactive receptor for one or more factor involved in promotion of neural sprouting when delivered to a neural cell in vivo. Preferably the receptors maintain high specific binding constants to said factor(s) and the biological activity of the receptor is reduced or absent. In a particularly preferred embodiment the transporter comprises some or all of a clostridial neurotoxin heavy chain, although other transporters such as the diphtheria toxin transporter may be effective in this regard as well.

In another embodiment the invention comprises a cholonergic specific transporter that is covalently or non-covalently joined to a nucleic acid which comprises a ribozyme or antisense nucleic acid able to specifically destroy the nucleic acids encoding neurotrophic agents or their receptors. Said joining may be made through methods including, but not limited to, covalent bonding or electrostatic forces.

In another embodiment, the present invention is drawn to stimulating the outgrowth of neural sprouts from damaged neural tissue. This could be effective in increasing the rate at which reinnervation occurs after a neural injury. This can be done with a composition comprising a polypeptide which comprises a neurotrophically active domain derived from an agent selected from a group consisting of IGF-1, IGF-2, cilary neurotrophic factor, NT-3, NT-4, brain derived neurotrophic factor, leukemia inhibitory factor, tenascin-C, ninjurin, neural cell adhesion molecule, and neural agrin. Such damage may be a result of neurotoxin poisoning or due to a traumatic event, including but not limited to nerve or spinal cord crush injuries, traumatic brain injuries, glaucoma-induced damage to the retina and/or optic nerve, or surgical trauma or injury.

The following examples illustrate various embodiments of the present invention, and are not intended to limit the scope of the invention, which is solely defined by the claims which conclude this specification.

### Example 1

Blepharospasm is a medical condition characterized by uncontrolled eyelid movement. In its early stages, the condition is characterized by excessive blinking or fluttering of the eyelids. The condition is generally a progressive one, in which excessive blinking is replaced in the later stages with spasms of eye closure that interfere with visual function. The spasms become more frequent and severe, and involve the preseptal, pretarsal, and orbicularis oculi muscle. The condition often results in functional blindness relatively quickly (in a matter of two to three years) after the symptoms are first encountered.

A patient suffering from moderate idiopathic blepharospasm is treated with injections of BoNT/A toxin preparation containing non-toxic proteins and hemagglutins in sterile saline. Alternatively, the same toxin preparation without hemagglutins may be used. The injections are generally in the volume of 100 µl; and each injection contains 1.25 to 2.5 units of the toxin preparation. The injections are made into the pretarsal orbicularis oculi of the upper lid laterally and medially and in the lower lid laterally and medially. Additionally, 2.5 unit injections (100 µl each) are made lateral to the lateral canthus and into the brow medially. Total amount of BoNT/A toxin injected is roughly 6.25 to 12.5 units per eye. The Bo/A toxin is provided in a sterile, preservative-free saline, and the same solution is used to dilute the BoNT/A toxin if the master preparation of it is too concentrated.

Following injection the treated muscles are sufficiently paralyzed due to this treatment to alleviate the major symptoms of blepharospasm. Some mild concomitant weakness in the surrounding muscle tissue is observed; these side effects are mild and tolerated well by the patient. The effect of this treatment lasts approximately 8 weeks, and must be repeated at the end of this time to maintain the beneficial effects.

### Example 2

A patient with blepharospasm is pre-treated with BoNT/A toxin as indicated in Example 1 with the following difference. Prior to injection with BoNT/A toxin preparation, the patient is given a ten-fold excess of IGFBP-4, having an amino acid sequence of SEQ ID NO: 1. The binding protein preparation is dissolved in sterile, preservative-free saline. Each injection is in the same area as the toxin injections that follow the pre-treatment; the volume of each injection is 100 µl. The BoNT/A toxin preparation is injected ten minutes after the injection of the IGFBT 4 injection.

The patient's therapeutic response to the BoNT/A toxin is similar to that seen in Example 1. The duration of the benefit provided by the BoNT/A toxin treatment is extended to 12 weeks or more, during which time no further injection need be made.

### Example 3

A patient with blepharospasm is pre-treated with BoNT/A toxin as indicated in Example 1 with the following difference. The BoNT/A toxin has been modified to have joined thereto a nucleic acid comprising a ribozyme specifically targeted to enzymatically destroy neural agrin mRNA. No supplemental injections are made.

The patient's therapeutic response to the BoNT/A toxin. is similar to that seen in Example 1. The duration of the benefit provided by the BoNT/A toxin treatment is extended to 12 weeks or more, during which time no further injection need be made.

### Example 4

A patient with blepharospasm is pre-treated with BoNT/A toxin as indicated in Example 1 with the following difference. The BoNT/A toxin has been modified to have joined thereto a nucleic acid encoding an inactive neurotrophin receptor which retains the ability to bind its target neurotrophin. No supplemental injections are made.

The patient's therapeutic response to the BoNT/A toxin is similar to that seen in Example 1. The duration of the benefit provided by the BoNT/A toxin treatment is extended beyond that seen with BoNT/A alone, during which time no further injection need be made.

It will be understood that, while reference is made to BoNT/A in the Examples above, any other of the species of botulinum toxins (e.g., BoNT/B through G) could be substituted therefor, with appropriate adjustments possibly necessary due to differences in specific activity of the toxin. Additionally, the light chain segment could be derived from any clostridial neurotoxin (or other neurotoxin) , with the heavy chain retaining the motor neuron receptor binding and exo-vescular transport activities retained from the BoNT heavy chain.

These Examples are not intended to exhaust the embodiments of the present invention, and the invention is not to be seen as limited thereby. Further embodiments will be disclosed within the claims that conclude this specification.

## Claims

1. Use of a composition effective in preventing the neuroregenerative activity of a polypeptide which composition comprises an agent selected from the group consisting of: IGF I, IGF II, cilary neurotrophic factor, NT-3, NT-4, brain-derived neurotrophic factor, leukemia inhibitory factor, tenascin-C, ninjurin, neural cell adhesion molecule, and neural agrin,
which agent is selected from the group consisting of:
a) an antibody able to selectively bind said polypeptide,
b) a competitive inhibitor of said polypeptide,
c) an enzyme cleaving a nucleic acid encoding said polypeptide,
d) an antisense agent able to selectively bind to a nucleic acid encoding said polypeptide,
e) a binding protein other than an antibody,
f) a ribozyme, and .
g) a nucleic acid encoding an inactive growth factor receptor able to bind said polypeptide,
for the preparation of a medicament for extending the effective period during which tissue treated with a clostridial toxin is paralyzed.

2. The use of Claim 1 wherein said agent is an antibody able to selectively bind said polypeptide.

3. The use of Claim 1 wherein said agent is a competitive inhibitor of said polypeptide.

4. The use of Claim 1 wherein said agent is an antisense agent able to selectively bind to a nucleic acid encoding said polypeptide.

5. The use of Claim 1 wherein said agent is a binding protein other than an antibody.

6. The use of Claim 5 wherein said polypeptide is selected from the group consisting of IGF I and IGF II, and said binding protein is selected from the group consisting of IGF-BP4 and IGF-BP5.

7. The use of any of the preceding Claims wherein said medicament is administered at the same time as said tissue is treated with said clostridial toxin.

8. The use of any of the preceding Claims wherein said medicament is administered prior to treatment of said tissue with said clostridial toxin.

9. The use of any of the preceding Claims wherein said clostridial toxin comprises BoNT.

10. The use of any of the preceding Claims wherein said clostridial comprises BoNT/A.

11. Use of a polypeptide which comprises the receptor binding and translocation activities of a clostridial toxin heavy chain, an active portion of a clostridial toxin light chain, and a neurotropically active domain derived from an agent selected from the group consisting of IGF I, IGF II, cilary neurotrophic factor, NT-3, NT-4, brain-derived neurotrophic factor, leukemia inhibitory factor, tenascin-C, ninjurin, neural cell adhesion molecule, and neural agrin, for the preparation of a medicament for stimulating the outgrowth of neural sprouts from damaged neural tissue.

12. Composition comprising a clostridial toxin and an agent effective in preventing the neuroregenerative , activity of a polypeptide selected from the group consisting of IGF I, IGF II, cilary neurotrophic factor, NT-3, NT-4, brain-derived neurotrophic factor, leukemia inhibitory factor,- tenascin-C, ninjurin, neural cell adhesion molecule, and neural agrin,
which agent is selected from the group consisting of:
a) an antibody able to'selectively bind said polypeptide,
b) a competitive inhibitor of said polypeptide,
c) an enzyme cleaving a nucleic acid encoding said polypeptide,
d) an antisense agent able to selectively bind to a nucleic acid encoding said polypeptide,
e) a binding protein other than an antibody,
f) a ribozyme, and
g) a nucleic acid encoding an inactive growth factor receptor able to bind said polypeptide.

13. The composition of Claim 12 wherein said polypeptide is selected from the group consisting of IGF I and IGF II and said binding protein other than an antibody is selected from the group consisting of IGF-BP4 and IGF-BP5.

## Patentansprüche

1. Verwendung einer Zusammensetzung, die für die Verhinderung einer neuroregenerativen Aktivität eines Polypeptids wirksam ist, wobei die Zusammensetzung ein Mittel umfasst, ausgewählt aus der Gruppe, bestehend aus IGF I, IGF II, ciliärem neurotrophem Faktor, NT-3, NT-4, von Hirn abgeleitetem neurotrophem Faktor, Leukämieinhibitionsfaktor, Tenascin-C, Ninjurin, neuralem Zelladhäsionsmolekül und neuralem Agrin,
wobei das Mittel ausgewählt ist aus der Gruppe, bestehend aus:
(a) einem Antikörper, der in der Lage ist, das Polypeptid selektiv zu binden,
(b) einem kompetitiven Inhibitor des Polypeptids,
(c) einem Enzym, das eine Nukleinsäure spaltet, die das Polypeptid codiert,
(d) einem Antisinn-Mittel, das in der Lage ist, eine Nukleinsäure selektiv zu binden, die das Polypeptid codiert,
(e) einem Bindeprotein, wobei es sich nicht um einen Antikörper handelt,
(f) einem Ribozym und
(g) einer Nukleinsäure, die einen inaktiven Wachstumsfaktorrezeptor codiert, der in der Lage ist, das Polypeptid zu binden,
für die Herstellung eines Medikaments für die Verlängerung der wirksamen Zeitspanne, während der mit einem Clostridientoxin behandeltes Gewebe paralysiert ist.

2. Verwendung gemäss Anspruch 1, wobei das Mittel ein Antikörper ist, der in der Lage ist, das Polypeptid selektiv zu binden.

3. Verwendung gemäss Anspruch 1, wobei das Mittel ein kompetitiver Inhibitor des Polypeptids ist.

4. Verwendung gemäss Anspruch 1, wobei das Mittel ein Antisinn-Mittel ist, das in der Lage ist, eine Nukleinsäure selektiv zu binden, die das Polypeptid codiert.

5. Verwendung gemäss Anspruch 1, wobei das Mittel ein Bindungsprotein ist, wobei es sich nicht um einen Antikörper handelt.

6. Verwendung gemäss Anspruch 5, wobei das Polypeptid ausgewählt ist aus der Gruppe, bestehend aus IGF I und IGF II, und wobei das Bindungsprotein ausgewählt ist aus der Gruppe, bestehend aus IGF-BP4 und IGF-BP5.

7. Verwendung gemäss einem der vorstehenden Ansprüche, wobei das Medikament zur selben Zeit verabreicht wird zu der das Gewebe mit dem Clostridientoxin behandelt wird.

8. Verwendung gemäss einem der vorstehenden Ansprüche, wobei das Medikament vor der Behandlung des Gewebes mit dem Clostridientoxin verabreicht wird.

9. Verwendung gemäss einem der vorstehenden Ansprüche, wobei das Clostridientoxin BoNT umfasst.

10. Verwendung gemäss einem der vorstehenden Ansprüche, wobei das Clostridientoxin BoNT/A umfasst.

11. Verwendung eines Polypeptids, das die Rezeptorbindungs- und Translokationsaktivitäten einer schweren Clostridientoxinkette umfasst, einen aktiven Teil einer leichten Kette eines Clostridientoxins und eine neurotroph aktive Domäne, abgeleitet von einem Mittel, ausgewählt aus der Gruppe, bestehend aus IGF I, IGF II, ciliärem neurotrophem Faktor, NT-3, NT-4, von Hirn abgeleitetem neurotrophem Faktor, Leukämieinhibitionsfaktor, Tenascin-C, Ninjurin, neuralem Zelladhäsionsmolekül und neuralem Agrin, für die Herstellung eines Medikaments für die Stimulierung des Wachstums neuraler Sprossen aus beschädigtem neuralem Gewebe.

12. Zusammensetzung, umfassend ein Clostridientoxin und ein Mittel, das wirksam ist zur Verhinderung der neuroregenerativen Aktivität eines Polypeptids, ausgewählt aus der Gruppe, bestehend aus IGF I, IGF II, ciliärem neurotrophem Faktor, NT-3, NT-4, von Hirn abgeleitetem neurotrophem Faktor, Leukämieinhibitionsfaktor, Tenascin-C, Ninjurin, neuralem Zelladhäsionsmolekül und neuralem Agrin,
wobei das Mittel ausgewählt ist aus der Gruppe, bestehend aus:
(a) einem Antikörper, der in der Lage ist, das Polypeptid selektiv zu binden,
(b) einem kompetitiven Inhibitor des Polypeptids,
(c) einem Enzym, das eine Nukleinsäure spaltet, die das Polypeptid codiert,
(d) einem Antisinn-Mittel, das in der Lage ist, eine Nukleinsäure selektiv zu binden, die das Polypeptid codiert,
(e) einem Bindeprotein, wobei es sich nicht um einen Antikörper handelt,
(f) einem Ribozym und
(g) einer Nukleinsäure, die einen inaktiven Wachstumsfaktorrezeptor codiert, der in der Lage ist, das Polypeptid zu binden.

13. Zusammensetzung gemäss Anspruch 12, wobei das Polypeptid ausgewählt ist aus der Gruppe, bestehend aus IGF I und IGF II, und wobei das Bindungsprotein, wobei es sich nicht um einen Antikörper handelt, ausgewählt ist aus der Gruppe, bestehend aus IGF-BP4 und IGF-BP5.

## Revendications

1. Utilisation d'une composition efficace pour la prévention de l'activité neuro-régénérative d'un polypeptide, laquelle composition comprend un agent sélectionné parmi le groupe constitué par : l'IGF I, l'IGF II, le facteur ciliaire neurotrophique, la NT-3, la NT-4, le facteur neurotrophique dérivé du cerveau, le facteur inhibiteur de leucémie (LIF), la ténascine-C, la ninjurine, la molécule d'adhésion cellulaire neurale et l'agrine neurale,
lequel agent est sélectionné parmi le groupe constitué par :
a) un anticorps capable de lier sélectivement ledit polypeptide,
b) un inhibiteur compétitif dudit polypeptide,
c) une enzyme clivant un acide nucléique codant pour ledit polypeptide,
d) un agent antisens capable de se lier sélectivement à un acide nucléique codant pour ledit polypeptide,
e) une protéine liante autre qu'un anticorps,
f) un ribozyme, et
g) un acide nucléique codant pour un récepteur de facteur de croissance inactif capable de lier ledit polypeptide,
pour la préparation d'un médicament permettant d'étendre la période efficace durant laquelle le tissu traité par une toxine clostridienne est paralysé.

2. L'utilisation selon la revendication 1, dans laquelle ledit agent est un anticorps capable de lier sélectivement ledit polypeptide.

3. L'utilisation selon la revendication 1, dans laquelle ledit agent est un inhibiteur compétitif dudit polypeptide.

4. L'utilisation selon la revendication 1, dans laquelle ledit agent est un agent antisens capable de se lier sélectivement à un acide nucléique codant pour ledit polypeptide.

5. L'utilisation selon la revendication 1, dans laquelle ledit agent est une protéine liante autre qu'un anticorps.

6. L'utilisation selon la revendication 5, dans laquelle ledit polypeptide est sélectionné parmi le groupe constitué par l'IGF I et l'IGF II, et ladite protéine liante est sélectionnée parmi le groupe constitué par l'IGF-BP4 et l'IGF-BP5.

7. L'utilisation selon l'une quelconque des revendications précédentes, dans laquelle ledit médicament est administré en même temps que ledit tissu est traité par ladite toxine clostridienne.

8. L'utilisation selon l'une quelconque des revendications précédentes, dans laquelle ledit médicament est administré avant le traitement dudit tissu par ladite toxine clostridienne.

9. L'utilisation selon l'une quelconque des revendications précédentes, dans laquelle ladite toxine clostridienne comprend la BoNT.

10. L'utilisation selon l'une quelconque des revendications précédentes, dans laquelle ladite clostridienne comprend la BoNT/A.

11. Utilisation d'un polypeptide qui comprend les activités de liaison au récepteur et de translocation d'une chaîne lourde de toxine clostridienne, une partie active d'une chaîne légère de toxine clostridienne et un domaine actif de manière neurotrope dérivé d'un agent sélectionné parmi le groupe constitué par l'IGF I, l'IGF II, le facteur ciliaire neurotrophique, la NT-3, la NT-4, le facteur neurotrophique dérivé du cerveau, le facteur inhibiteur de leucémie (LIF), la ténascine-C, la ninjurine, la molécule d'adhésion cellulaire neurale, et l'agrine neurale pour la préparation d'un médicament destiné à stimuler l'excroissance de bourgeons neuraux à partir de tissu neural endommagé.

12. Une composition comprenant une toxine clostridienne et un agent efficace pour la prévention de l'activité neuro-régénérative d'un polypeptide sélectionné parmi le groupe constitué par l'IGF I, l'IGF II, le facteur ciliaire neurotrophique, la NT-3, la NT-4, le facteur neurotrophique dérivé du cerveau, le facteur inhibiteur de leucémie (LIF), la ténascine-C, la ninjurine, la molécule d'adhésion cellulaire neurale et l'agrine neurale, lequel agent est sélectionné parmi le groupe constitué par
a) un anticorps capable de lier sélectivement ledit polypeptide,
b) un inhibiteur compétitif dudit polypeptide,
c) une enzyme clivant un acide nucléique codant pour ledit polypeptide,
d) un agent antisens capable de se lier sélectivement à un acide nucléique codant pour ledit polypeptide,
e) une protéine liante autre qu'un anticorps,
f) un ribozyme, et
g) un acide nucléique codant pour un récepteur de facteur de croissance inactif capable de lier ledit polypeptide,

13. La composition de la revendication 12, dans laquelle ledit polypeptide est sélectionné parmi le groupe constitué par l'IGF I et l'IGF II et ladite protéine liante autre qu'un anticorps est sélectionnés parmi le groupe constitué par l'IGF-BP4 et l'IGF-BP5.
